(19) **Europäisches Patentamt**

**European Patent Office**

**Office européen des brevets**

(11) Veröffentlichungsnummer: **0 244 789**
**A2**

(12) # EUROPÄISCHE PATENTANMELDUNG

(21) Anmeldenummer: **87106383.0**

(22) Anmeldetag: **02.05.87**

(51) Int. Cl.⁴: **G01N 21/51** , G01N 33/28

(30) Priorität: **06.05.86 DE 3615222**

(43) Veröffentlichungstag der Anmeldung:
**11.11.87 Patentblatt 87/46**

(84) Benannte Vertragsstaaten:
**BE CH DE FR GB LI NL**

(71) Anmelder: **HOECHST AKTIENGESELLSCHAFT**
**Postfach 80 03 20**
**D-6230 Frankfurt am Main 80(DE)**

(72) Erfinder: **Miller, Dennis, Dr.**
**Waldallee 73**
**D-6239 Eppstein/Taunus(DE)**

(54) **Vorrichtung zur Messung der Lichtstreuung in stark streuenden dispersen Systemen.**

(57) Vorrichtung zur Messung der Lichtstreuung in stark streuenden dispersen Medien. Bei dieser Meßvorrichtung wird aus einer Lichtquelle, bevorzugt aus einem He-Ne Laser Licht in eine Meßzelle gestrahlt, in der sich das zu untersuchende Medium befindet. Das aus der Küvette austretende Licht wird mit einem Lichtdetektor (Photomultiplier) gemessen. Um den Einfluß von Fremdlicht auszuschalten, ist es vorteilhaft, dem Laser einen Chopper nachzuschalten und das am Photomultiplier empfangene Meßsignal in einem Lock-in Verstärker mit angeschlossenem Rechner und Schreiber am Ausgang des Photomultipliers zu verarbeiten.

FIG.1

FIG.2

EP 0 244 789 A2

## Vorrichtung zur Messung der Lichtstreuung in stark streuenden dispersen Systemen

Für schwach streuende Flüssigkeiten sind Lichtstreumethoden sowohl theoretisch als auch apparativ sehr weit entwickelt. Sie ermöglichen Aussagen über die Größe und Form der dispergierten Partikel. Für die Vermessung von stark trüben Systemen sind dagegen in der Literatur nur wenige Arbeiten zu finden. Besondere Bedeutung hat die Messung der Lichtstreuung bei den stark trüben Rohölen, die Wasser-in-Öl Emulsionen darstellen. Bei herkömmlichen Messungen der Lichtstreuung bzw. Trübung verursacht die starke Lichtabsorption noch große Schwierigkeiten. Bei der Aufbereitung von Rohölen werden diese Emulsionen durch Zugabe eines sogenannten Spalters oder Demulgators gebrochen und so die Ölphase von der Wasserphase getrennt. Dieser Vorgang wird durch eine Koaleszenz der Wassertröpfchen eingeleitet und für die Bestimmung der Effektivität der verschiedenen Rohöl-Spalter istr es von Bedeutung, diese Koaleszenz in Rohölen nach Zugabe eines Spalters zu untersuchen.

Es wurde nun eine Vorrichtung gefunden, die es gestattet, solche Koaleszenzvorgänge in stark streuenden dispersen Systemen, insbesondere in Rohölemulsionen, mit Hilfe der Lichtstreuung zu untersuchen.

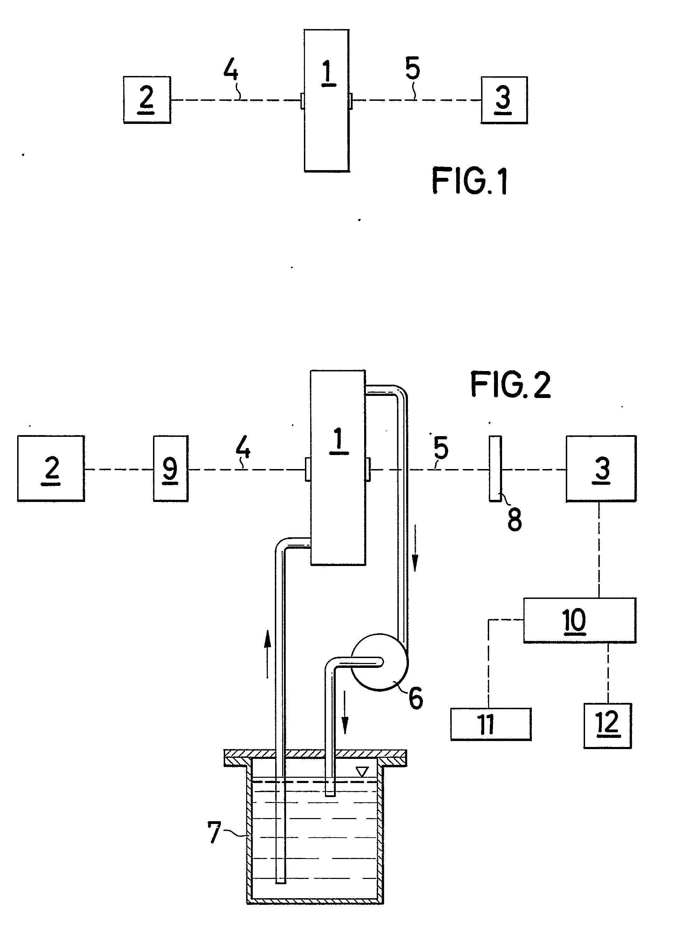

Eine zur Durchführung dieser Messung geeignete Vorrichtung ist schematisch in Figur 1 dargestellt. Sie besteht im wesentlichen aus einer Küvette aus Glas (1), einer Lichtquelle (2) und einem Lichtdetektor (3). Von der Lichtquelle und dem Lichtdetektor gehen Lichtleiter (4) und (5) aus, deren Enden auf zwei gegenüberliegenden Flächen der Küvette aufliegen. Die Enden der Lichtleiter stehen sich genau gegenüber.

Eine bevorzugte Ausführungsform hierzu ist in Figur 2 dar gestellt. Die Glasküvette (1) ist über die Pumpe (6) mit einem Vorratsgefäß (7) verbunden, in dem sich die zu untersuchende Emulsion befindet und in das der zu untersuchende Spalter gegeben werden kann. Zweckmäßigerweise wird dieses Vorratsgefäß durch hier nicht dargestellte Heiz-und Kühlvorrichtungen und mittels eines Thermostats auf konstanter Temperatur gehalten. Außerdem ist das Vorratsgefäß noch mit einem Rührer versehen. Auf der einen Seite der Küvette (1) liegt das Ende eines Lichtleiters (4) auf, der von der Lichtquelle (2), hier ein He-Ne-Laser mit einer Leistung von 5 milli-Watt, ausgeht. Auf der gegenüberliegenden Seite der Küvette, genau gegenüber dem Endpunkt des von der Lichtquelle ausgehenden Lichtleiters liegt das Ende eines zweiten Lichtleiters (5) auf, der zu einem Photomultiplier (3) führt, der als Lichtdetektor fungiert. Die Küvette selbst hat eine Dicke von ca. 0,3 bis 3 mm, vorzugsweise 1 mm.

Zweckmäßigerweise befindet sich zwischen Küvette und Photomultiplier ein Interferenzfilter (8), durch das ein Einfluß von Fremdlicht vermieden werden soll. Gegebenenfalls kann man diesen Interferenzfilter auch mit einem Graufilter kombinieren, wenn die Intersität des aus der Küvette austretenden Lichts zu groß ist für den Lichtdetektor. Der Unterdrückung des Fremdlichts dient auch die Kombination von Chopper (9) und Lock-in Verstärker (10), wobei der Chopper zwischen Laser (2) und Glasküvette (1) und der Lock-in Verstärker am Ausgang des Photomultipliers angeordnet ist. Das am Lock-in Verstärker erhaltene Meßsignal wird durch einen Rechner (11) und Schreiber (12) ausgewertet.

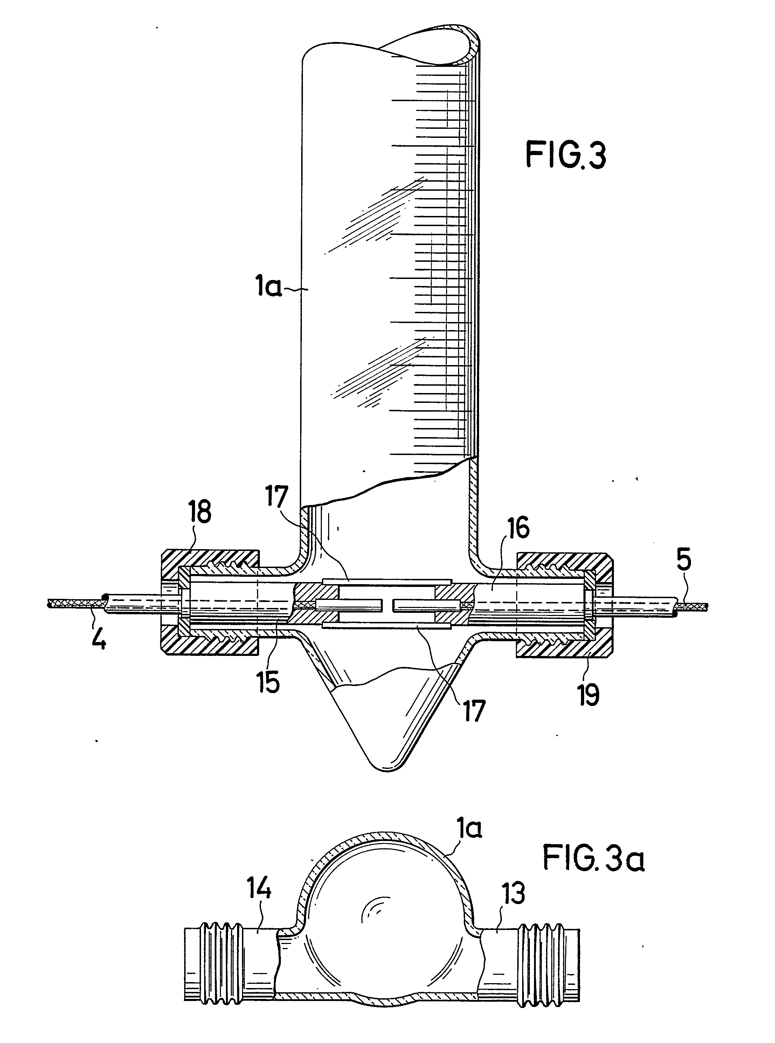

Eine andere Ausführungsform für eine geeignete Meßzelle ist in Figur 3 und 3a dargestellt. Sie besteht aus einem unter konisch geschlossenen Glasgefäß (1a) mit zwei kreisförmigen, sich horizontal gegenüberliegenden Öffnungen (13) und (14), zwei in diesen Öffnungen eingelagerten zylinderförmigen Halterungen (15) und (16), die jeweils in ihrer Achse einen Lichtleiter (4) bzw. (5) tragen, wobei die beiden sich gegenüberliegenden Enden dieser Halterungen in geeigneter Weise starr überbrückt sind, so daß die Enden der beiden Lichtleiter genau gegenüberstehen. Die sich gegenüberliegenden Enden der beiden Halterungen sind am einfachsten durch ein oder mehrere fest montierte Stege (17) oder auf andere Weise starr miteinander verbunden, damit die beiden Enden der Lichtleiter im gewünschten Abstand voneinander fest in ihrer Position fixiert werden. Dieser Abstand der beiden Lichtleiter soll ca. 0,3 bis 3 mm betragen. Die beiden Halterungen für die Lichtleiter werden in den Öffnungen (13) und (14) durch aufgeschraubte Kappen (18) und (19) festgehalten. Die beiden Öffnungen (13) und (14) sind vorzugsweise so angeordnet, wie aus Figur 3a hervorgeht, d.h. die Mittelachse der Öffnungen (13) und (14) ist auf der horizontalen Schnittebene etwas seitlich verschoben, so daß Raum bleibt für einen von oben in die Meßzelle eingeführten Rührer. Die übrige Anordnung der Apparaur ist die gleiche wie oben bei Figur 2 beschrieben, d.h. die dort beschriebene Küvette mitsamt dem Vorratsgefäß und Pumpe für die zu messende Flüssigkeit wird gegen die in Figur 3 beschriebene Meßzelle ausgetauscht.

Bei all diesen Anordnungen wird das in die Meßzelle eingestrahlte Licht in dem dispersen Medium gestreut und aus der Abschwächung des Meßsignals wird die effektive optische Dichte D gemäß der Formel $D = \log \frac{I_0}{I} \cdot l^{-1}$ ermittelt. I ist der Meßwert des untersuchten dispersen Systems, Io ist der Meßwert in einer transparenten Flüssigkeit, z. B. Wasser und l ist der Lichtweg in dem dispersen Medium. Auf diese Weise kann man sehr gut die Koaleszenzwirkung verschiedener Erdölspalter untersuchen, denn abhängig von der Wirksamkeit der Spalter zeigt sich mit der geschilderten Meßvorichtung eine mehr oder weniger steile Verringerung der optischen Dichte.

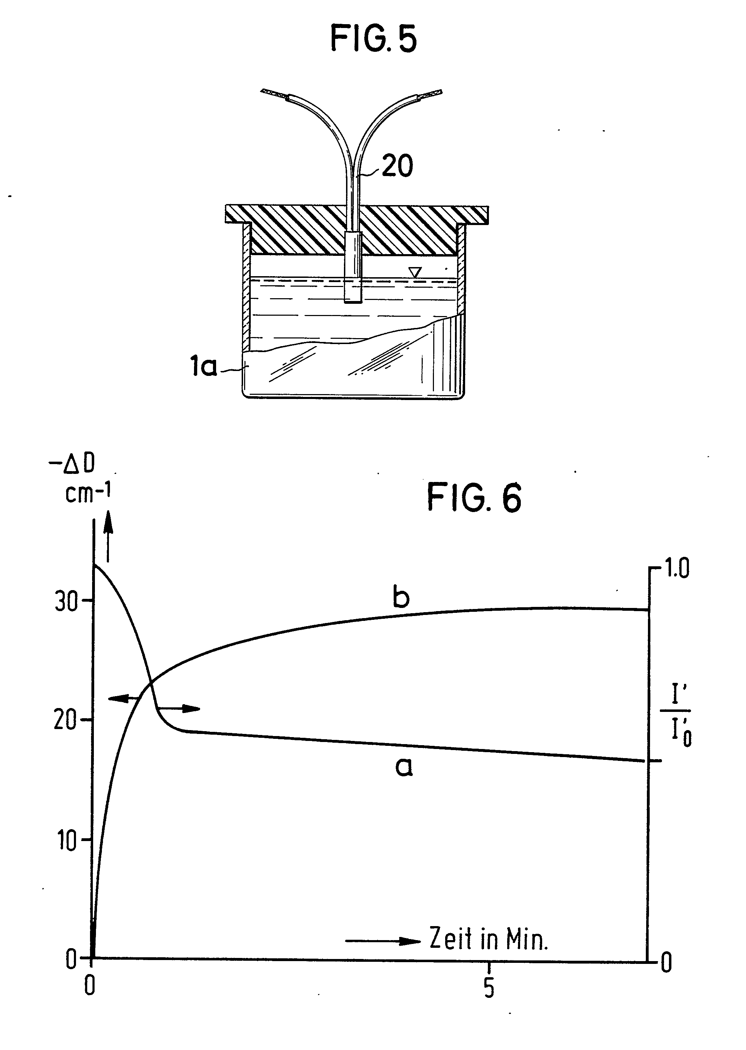

Man kann die Lichtstreuung auch über das rückwärts ge streute Licht messen. Dies geschieht zweckmäßigerweise mit einem Reflexlichtleiter, dessen Sender-und Empfängerfasern statistisch gemischt sind. Eine geeignete Ausführungsform ist in Figur 5 dargestellt. Sie besteht aus einem Glasgefäß (1a) mit einem Deckel, in dem der Reflexlichtleiter (20) montiert ist. Das Gefäß wird durch einen Magnetrührer gerührt. Die übrige Anordnung der Apparatur ist die gleiche wie oben bei Figur 2 beschrieben, d.h. die dort beschriebene Küvette mitsamt dem Vorratsgefäß, Pumpe und Lichtleiter wird gegen das in Figur 5 beschriebene Meßgefäß ausgetauscht. Gemessen wird die Lichtintensität l'. Die Lichtintensität vor Zugabe des Demulgators wird mit lo' bezeichnet. Das Ausmaß der Koaleszenz kann durch den zeitlichen Verlauf des Verhältnisses l'/lo' abgeschätzt werden.

Beispiel 1

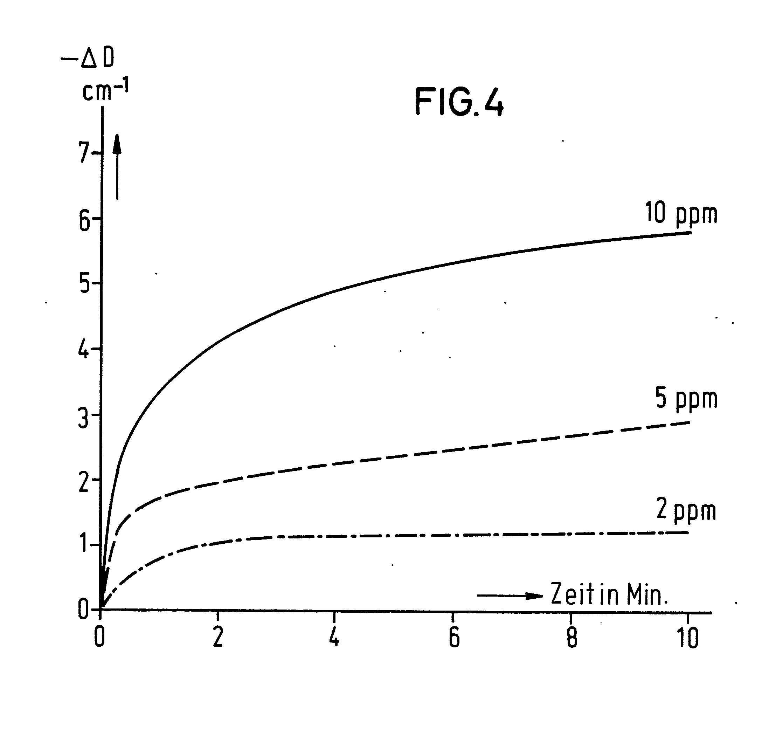

In einer Apparatur wie in Figur 2 dargestellt, wurde die Koaleszenzwirkung unterschiedlicher Mengen eines Propylenoxid-Ethylenoxid-Blockpolymers auf der Basis eines Propylenoxids mit einem Molgewicht von 2000, umgesetzt mit 38 % Ethylenoxid, bei einer Emulsion bestehend aus 82 % Rohöl aus Norddeutschland und 18 % Wasser untersucht. Die Temperatur betrug 40°C. Der Demulgator wurde als verdünnte Lösung in Xylol zu der Zeit t = 0 zudosiert. Wie in Fig. 4 zu sehen, zeigt sich schon nach wenigen Sekunden eine Zunahme der Lichtdurchlässigkeit bzw. eine Abnahme der effektiven optischen Dichte -ΔD infolge der Koaleszenz. Mit zunehmenden Mengen an Demulgator beobachtet man eine stärkere Koaleszenz.

Beispiel 2

Die Untersuchung der Koaleszenz durch Messung des rückwärts gerichteten Lichts und durch Lichtabschwächung wurden verglichen. Für die Messungen der Lichtabschwächung wurde eine Meßzelle wie in Figur 3 benutzt, für die Messungen aus rückwärts gestreutem Licht eine Anordnung wie in Figur 5 benutzt, Die Emulsion bestand aus gleichen Anteilen wasserfreiem süddeutschem Rohöl und 0,5 % NaCl-Lösung. Der Demulgator war ein oxethyliertes Alkylphenolformaldehydharz;

es wurden 40 ppm des Demulgators als verdünnte Lösung in Xylol zudosiert. Die Ergebnisse sind in Figur 6 dargestellt. Kurve a zeigt die Meßwerte für die Messung der Lichtstreuung mittels des Reflexlichtleiters, Kurve b zeigt die Werte, für die Änderung der Lichtdeurchlässigkeit, wie sie mit einer Meßzelle nach Figur 3 erhalten werden. Wie erwartet korreliert eine Zunahme der Lichtdurchlässigkeit mit einer Abnahme des rückwärts gestreuten Lichtes. Man sieht, daß beide Meßanordnungen benutzt werden können, um die Änderung der Lichtstreuung infolge Koaleszenz zu untersuchen.

## Ansprüche

1. Vorrichtung zur Messung der Lichtstreuung in stark streuenden dispersen Medien bestehend aus einer Glasküvette, einer Lichtquelle und einem Lichtdetektor, sowie zwei Lichtleitern, die von der Lichtquelle und dem Lichtdetektor ausgehen und deren Enden auf der Küvette aufliegen und sich genau gegenüberstehen.

2. Vorrichtung zur Messung der Lichtstreuung in stark streuenden dispersen Medien bestehend aus einer Meßzelle in Form einer Glasküvette die über eine Pumpe mit einem Vorratsgefäß verbunden ist, einer He-Ne Laser Lichtquelle mit nachgeschaltetem Chopper, einem Photomultiplier als Lichtdetektor mit vorgeschaltetem optischem Filter, wobei von dem Chopper und dem optischen Filter jeweils ein Lichtleiter ausgeht, deren Enden auf der Küvette aufliegen und sich genau gegenüberstehen, sowie einem Lock-in Verstärker mit angeschlossenem Rechner und Schreiber am Ausgang des Photomultipliers.

3. Vorrichtung nach Anspruch 1, dadurch gekennzeichnet, daß die Meßzelle besteht aus einem unten konisch geschlossenen Glasgefäß mit zwei kreisförmigen, sich horizontal gegenüberliegendne Öffnungen, die in diesen Öffnungen eingelagerten zylinderförmigen Halterungen, die jeweils in ihrer Achse einen Lichtleiter tragen, wobei die beiden sich gegenüberliegenden Enden dieser Halterungen starr überbrückt sind.

4. Vorrichtung nach Anspruch 2, dadurch gekennzeichnet, daß die Meßzelle aus einem Glasgefäß besteht, in dessen Deckel ein Reflexlichtleiter montiert ist.

FIG.1

FIG.2

FIG.3

1a

17

18

16

5

4

15

19

17

FIG.3a

1a

14

13

FIG.4

# FIG. 5

# FIG. 6